# EUROPÄISCHE PATENTSCHRIFT

(11) **EP 1 475 634 B1**
(45) Veröffentlichungstag und Bekanntmachung des Hinweises auf die Patenterteilung: **14.03.2007**
(21) Anmeldenummer: 03008841.3
(22) Anmeldetag: 25.04.2003
(51) Int. Cl.: G01N 29/26, A61B 8/14, G01S 15/89, G01S 15/06, G01S 7/539, G06T 7/60, G01R 33/28

(54) **Verfahren und Vorrichtung zur Bildoptimierung bei Ultraschallaufnahmen**
Method and apparatus for optimization of ultrasonic imaging
Procédé et dispositif pour optimiser de l'imagerie ultrasonore

(43) Veröffentlichungstag der Anmeldung: 10.11.2004
(73) Patentinhaber: BrainLAB AG, 85551 Kirchheim/Heimstetten (DE)
(72) Erfinder: Vilsmeier, Stefan, 6330 Kufstein (AT); Pfäffle, Alexander, 85435 Erding (DE); Schmidt, Robert, 81371 München (DE)
(74) Vertreter: Schwabe - Sandmair - Marx

(56) Entgegenhaltungen:
- EP-A- 0 985 380
- EP-A- 1 152 372
- EP-A- 1 264 577
- US-A- 6 138 495
- US-B1- 6 423 006

## Beschreibung

Die vorliegende Erfindung bezieht sich auf Verfahren und Vorrichtungen zur Bildoptimierung bei Ultraschallaufnahmen. Insbesondere bezieht sich die Erfindung auf Verfahren und Vorrichtungen zum automatischen Einstellen eines Ultraschallgeräts und zum Positionieren einer Ultraschall-Sonde.

Moderne Ultraschallgeräte haben eine Vielzahl von Abbildungs-Modi mit einer Vielzahl einzustellender Parameter. So hat zum Beispiel das von SIEMENS (R) hergestellte SONOLINE Omnia Ultraschallsystem, welches für die Erfindung prinzipiell verwendet werden kann, Bildgebungsfunktionen im B-Mode und M-Mode, wobei zum Beispiel verschiedene Doppler-Funktionen und Farb-Doppler-Bildgebungsverfahren eingestellt werden können. In der Bildgebungsart B-Mode (B für "brightness" (Helligkeit)) wird ein aufgenommenes Bild in zweidimensionaler Graustufendarstellung erzeugt, wobei die Helligkeit eines Pixels des erzeugten Bildes von der Intensität des reflektierten Schalls abhängt. Im Farb-Doppler-Betrieb wird einem definierten Fenster oder einem interessierenden Bereich (ROI = Range of Interest) im B-Bild eine Farbe zugeordnet, wobei die Tiefe, die Lage und die Größe des Fensters, sowie verschiedene sich auf die Farbgebung beziehenden Parameter eingestellt werden können, um zum Beispiel die Bewegung einer Flüssigkeit, wie zum Beispiel Blut, oder Gewebe bildlich darzustellen. Somit kann zum Beispiel der Blutstrom im Herz oder in Gefäßen unter Verwendung des Doppler-Effekts gemessen werden.

Jedoch muss eine Vielzahl von Parametern an einem Ultraschallgerät manuell eingestellt werden, um einen interessierenden Bereich, wie zum Beispiel Knochen, Gefäße oder einen bestimmten Gewebebereich zu fokussieren. Diese Einstellung eines Ultraschallsystems ist relativ zeitaufwendig und kann nur mit entsprechender Erfahrung durchgeführt werden, so dass nicht immer die optimal erzielbare Bildqualität eines interessierenden Bereichs erzeugt werden kann. Beispiele für einzustellende Parameter eines Ultraschall-Gerätes sind die Schalttiefe, der Tiefenausgleich (depth gain compensation), der Dynamikbereich, Kontur, Fokus, Bildrate oder Zeilendichte, Frequenz, Verstärkung, Grauskalen, die Sendeleistung, Nachbearbeitungskurven zur Optimierung der Graustufen, die Einstellung des Sektorwinkels oder der Bildbreite oder die Einstellung einer Vergrößerung (Zoom). Weitere Einstellungen sind zum Beispiel für die Optimierung von dreidimensionalen Bildern vorzunehmen.

Aus der US 6,322,509 B1 und dem darin beschriebenen Stand der Technik sind verschiedene Verfahren zum Einstellen von Parametern von Ultraschallgeräten bekannt. Bezüglich der allgemeinen Funktionsweise von Ultraschallgeräten und bezüglich der an Ultraschallgeräten einzustellenden Parameter wird auf die US 6,322,509 B1, sowie auf die Bedienungsanleitung des SONOLINE Omnia Ultraschallsystems von SIEMENS verwiesen, deren Lehren bezüglich der Funktionsweise von Ultraschallgeräten und bezüglich der an einem Ultraschallgerät einzustellenden Parameter in diese Anmeldung aufgenommen werden.

EP 1 264 577 A1 offenbart eine bildgebende Vorrichtung, wobei mindestens zwei verschiedene bildgebende Verfahren verwendet werden, um eine verbesserte Darstellung eines untersuchten Objektes zu erhalten, wobei beispielsweise ein Kernspintomographiebild in ein Ultraschallbild integriert wird.

Es ist eine Aufgabe der vorliegenden Erfindung Verfahren und Vorrichtungen zur Optimierung der von Ultraschallgeräten erzeugten Bilder vorzuschlagen.

Diese Aufgabe wird durch die Gegenstände der unabhängigen Ansprüche gelöst. Vorteilhafte Ausführungsformen ergeben sich aus den Unteransprüchen.

Gemäß einer ersten Ausführungsform, welche in Anspruch 1 definiert ist, bezieht sich die Erfindung auf ein Verfahren zum Einstellen oder Festlegen mindestens eines Einstellparameters einer Ultraschallvorrichtung, wobei das Objekt oder der Körper, von welchem ein Ultraschallbild mindestens eines Teilbereichs dieses Objektes erzeugt werden soll, durch ein bevorzugt bildgebendes Verfahren, wie zum Beispiel eine Kernspinresonanz (MRI)-Aufnahme, Computertomografie, PET, SPECT oder eine Röntgenaufnahme bzw. Fluoroskopie aufgenommen wird. Solche Aufnahmeverfahren sind im Stand der Technik bekannt. Besonders vorteilhaft wird ein bildgebendes Aufnahmeverfahren durchgeführt, das nicht auf Ultraschallaufnahmen basiert, da durch die oben erwähnten Aufnahmeverfahren die dreidimensionale Struktur eines Objekts oder Körpers relativ einfach ermittelt werden kann. Aus den so gewonnenen Daten des Objektes oder eines Teilbereichs davon werden Daten, wie zum Beispiel Bilddaten erzeugt, welche erfindungsgemäß die Grundlage zum objekt- oder körperspezifischen Einstellen mindestens eines Einstellparameters der Ultraschall-Vorrichtung sind. Beispielsweise kann durch eine MRI-Aufnahme ermittelt werden, wo ein interessierender Bereich (ROI) im Objekt liegt, so dass beispielsweise die Sendeleistung, der Tiefenausgleich, die Verstärkung und/oder weitere Parameter der Ultraschall-Vorrichtung, wie in Anspruch 1 definiert, so eingestellt werden können, dass der interessierende Bereich mit einer möglichst guten oder optimalen Bildqualität dargestellt wird.

Soll beispielsweise der Verlauf von Blutgefäßen, die Lage eines erkrankten Gewebes wie zum Beispiel eines Tumors, oder die Position von Knochen in einem Körper durch das Ultraschallgerät dargestellt werden, so können durch eine vorherige Aufnahme mit einem bildgebenden Verfahren Informationen bezüglich der abzubildenden Struktur im Körper gewonnen werden, wie zum Beispiel der Abstand einer Struktur oder des interessierenden Bereichs von der Oberfläche des Körpers oder der Verlauf der Struktur im Körper, aus welchen Einstellungen für Parameter der Ultraschall-Vorrichtung ermittelt oder errechnet werden können, um den interessierenden Bereich (ROI) mit möglichst guter Bildqualität darzustellen. Somit ist ein manuelles Einstellen und Nachjustieren der Ultraschall-Vorrichtung nicht erforderlich, da zum Beispiel alle zur Einstellung der Ultraschall-Vorrichtung benötigten Parameterwerte aus der erfindungsgemäß vorher durchgeführten Aufnahme des Objektes oder des zu untersuchenden Körpers ermittelt werden können. Damit ist eine automatische körperspezifische Initialisierung und/oder Einstellung einer Ultraschallvorrichtung möglich.

Bei einem Verfahren zum Positionieren mindestens einer Ultraschall-Sonde an einem zu untersuchenden Körper oder Objekt, wird wie oben beschrieben das Objekt durch ein bevorzugt bildgebendes Verfahren aufgenommen und aus den Aufnahmedaten werden zum Beispiel Bilddaten erzeugt, welche Informationen zur Verfügung stellen, wo eine oder mehrere Ultraschall-Sonden besonders vorteilhaft oder möglichst optimal zu positionieren sind, damit ein oder mehrere Ultraschall-Bilder mit möglichst guter Qualität erzeugt werden können. Die Information bezüglich der möglichst optimalen Position mindestens einer Ultraschall-Sonde kann zum Beispiel in Form einer absoluten Größe ausgegeben werden, beispielsweise als Information, dass diese Position 4 cm oberhalb des Bauchnabels liegt. Ebenso ist es möglich, dass bekannte Navigations- und Tracking-Verfahren verwendet werden, um eine Sonde möglichst optimal an dem zu untersuchenden Objekt oder Körper zu positionieren. Hierzu können auf bekannte Weise beispielsweise reflektierende Marker an der mindestens einen Ultraschall-Sonde und an dem zu untersuchenden Objekt oder Körper angebracht werden, wodurch es möglich ist die Sonde relativ zum Körper zu navigieren und an der gewünschten optimalen Stelle zu positionieren.

Vorteilhaft können die beiden oben beschriebenen Verfahren, nämlich das Generieren mindestens eines Einstellwertes für mindestens einen Einstellparameter der Ultraschallvorrichtung mit dem möglichst vorteilhaften Positionieren mindestens einer Ultraschall-Sonde kombiniert werden, wobei für diese Verfahren jeweils auf Daten zurückgegriffen wird, welche durch die Aufnahme des durch die Ultraschallvorrichtung zu untersuchenden Objekts durch ein bevorzugt bildgebendes Verfahren zur Verfügung gestellt wurden.

Die Ultraschall-Vorrichtung wird so eingestellt, dass in Abhängigkeit von der Position der Ultraschall-Sonde und basierend auf den zur Position korrespondierenden Bilddaten Parameter der Ultraschall-Vorrichtung so gewählt werden, dass zu dieser Position der Ultraschall-Sonde ein möglichst optimales Ultraschall-Bild generiert werden kann.

Insbesondere ist es vorteilhaft die Parameter zur Einstellung der Ultraschall-Vorrichtung so zu wählen oder so zu verändern, dass zu einer zum Beispiel durch ein Navigationssystem ermittelten Position einer Ultraschall-Sonde an einem zu untersuchenden Objekt durch Aufnahmedaten des Objekts eine Einstellung der Ultraschall-Vorrichtung erhalten wird, welche es ermöglicht, dass ein interessierender Bereich mit möglichst guter Bildqualität dargestellt wird. Wird zum Beispiel durch das Navigationssystem eine Positionsveränderung oder Verschiebung einer Ultraschall-Sonde relativ zu einem zu untersuchenden Objekt erkannt, ist es erfindungsgemäß möglich die Einstellparameter der Ultraschall-Vorrichtung automatisch so zu verändern, dass ein interessierender Bereich immer gut dargestellt werden kann.

Es können bevorzugt Daten vom Ultraschall-Gerät ausgegeben werden, welche zur Positionierung der Ultraschall-Sonde verwendet werden können. Beispielsweise kann anhand der durch eine Ultraschall-Sonde aufgenommenen Bilder ermittelt werden, ob die Sonde richtig positioniert ist, oder ob die Sonde noch in eine bestimmte Richtung bewegt werden muss. Dabei können beispielsweise durch die von der Sonde aufgenommenen Bilddaten Informationen gewonnen werden, wie die Sonde bewegt oder navigiert werden muss, um richtig positioniert zu sein.

In einem weiteren, nicht beanspruchtem Ausführungsbeispiel wird zur Ermittlung von Werten der Einstellparameter zur Abbildung eines gewünschten Bereichs auf spezifische objektabhängige oder objekttypische Informationen bezüglich der gewünschten abzubildenden Bereiche zurückgegriffen. Beispielsweise kann ein erster Parametersatz vorgegeben werden, welcher eine vorteilhafte Einstellung der Ultraschall-Vorrichtung zur Darstellung von Blutgefäßen ermöglicht und ein zweiter Parametersatz kann vorgegeben werden, welcher vorteilhaft zur Darstellung von Knochen, bestimmten Gewebestrukturen wie z.B. Tumoren oder anderen von einem Benutzer wählbaren interessierenden Strukturen ist. Ebenso können weitere Parametersätze zur Darstellung von jeweils interessierenden Bereichen oder Körperstrukturen vorgegeben werden, welche die Erzeugung eines Bildes eines bestimmten interessierenden Bereichs oder Gewebes mit möglichst guter Qualität ermöglichen.

Weiterhin bezieht sich die Erfindung auf ein Computerprogramm, welches, wenn es in einen Computer geladen wird, oder auf einem Computer läuft, mindestens einen der oben beschriebenen Verfahrensschritte ausführt. Des weiteren bezieht sich die Erfindung auf ein Programmspeichermedium oder Computerprogrammprodukt mit einem solchen Programm.

Gemäß einem weiteren Aspekt bezieht sich die Erfindung auf eine Vorrichtung gemäß Anspruch 4 zum Einstellen mindestens eines Einstellparameters einer Ultraschall-Vorrichtung zur Erzeugung einer Ultraschallaufnahme eines Objektes mit einer Aufnahmevorrichtung zur Aufnahme des Objekts. Vorteilhaft kann mit der Aufnahmevorrichtung ein bildgebendes Verfahren durchgeführt werden, um das Objekt zu untersuchen. Erfindungsgemäß verwendbare Aufnahmevorrichtungen sind Vorrichtungen, welche sich zur Durchführung der oben beschriebenen Aufnahmeverfahren, wie zum Beispiel MRI, CT, PET, SPECT oder Fluoroskopie eignen. Weiterhin weist die erfindungsgemäße Vorrichtung eine Auswerte- oder Recheneinheit auf, welche aus den Aufnahmedaten des Objekts mindestens eine Einstellung eines Einstellparameters der Ultraschall-Vorrichtung ermitteln kann, um zum Beispiel einen gewünschten vorgegebenen Bereich möglichst gut durch die Ultraschall-Vorrichtung darstellen oder abbilden zu können.

Eine Vorrichtung zum Positionieren mindestens einer Ultraschall-Sonde hat mindestens eine wie oben beschriebenen Aufnahmevorrichtung und eine Auswerte- oder Recheneinheit, welche eine Position für die Ultraschall-Sonde an dem durch die Ultraschall-Vorrichtung zu untersuchenden Körper oder Objekt aus den Aufnahme-Daten ermittelt, so dass mindestens eine Ultraschall-Sonde möglichst optimal an oder auf dem zu untersuchenden Körper oder Objekt positioniert werden kann.

Ebenso wie oben beschrieben ist es möglich beide Vorrichtungen zu kombinieren, so dass aus Aufnahme-Daten eines zu untersuchenden Objekts oder Körpers, welche durch bildgebende Verfahren gewonnen wurden, mindestens ein Wert eines Einstellparameters ermittelt werden kann und eine Position für mindestens eine Ultraschall-Sonde ermittelt werden kann.

Vorteilhaft ist bei einer erfindungsgemäßen Vorrichtung ein Tracking- oder Navigationssystem vorgesehen, um mindestens eine Sonde möglichst präzise an eine gewünschte Position an oder auf dem zu untersuchenden Objekt bringen zu können.

In einem weiteren, nicht beanspruchtem Ausführungsbeispiel ist ein Speicher vorgesehen, in welchem Parametersätze zum Einstellen einer Ultraschall-Vorrichtung gespeichert sind, um zum Beispiel eine Ultraschall-Vorrichtung so zu initialisieren oder einzustellen, dass diese zur Abbildung von bestimmten Strukturen oder von einem bestimmten Gewebe, wie zum Beispiel Gefäßen oder Muskeln optimiert ist. Vorteilhaft können diese Parametersätze verwendet werden, um zusammen mit erfindungsgemäß objekt- oder körperspezifisch ermittelten Daten eine optimale Einstellung einer Ultraschall-Vorrichtung zur Abbildung eines bestimmten Bereiches zu erhalten.

Weiterhin bezieht sich die Erfindung auf ein System mit mindestens einer wie oben beschriebenen Vorrichtung und einer Ultraschall-Vorrichtung, welche bevorzugt mit dieser mindestens einen Vorrichtung über ein Interface oder eine Schnittstelle gekoppelt ist, um zum Beispiel Parameter zur Einstellung der Ultraschall-Vorrichtung, Bilddaten oder Positionsdaten zur Anordnung mindestens einer Ultraschall-Sonde zu übertragen.

Die Erfindung wird nachfolgend anhand eines bevorzugten Ausführungsbeispiels unter Bezugnahme auf die beiliegende Figur beschrieben. Es zeigt:

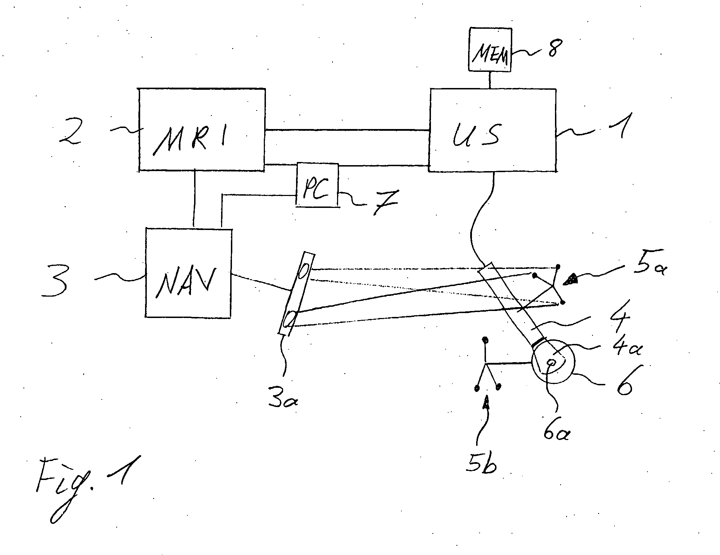

- Figur 1: Eine Ausführungsform der erfmdungsgemäßen Vorrichtung, mit welcher ein erfindungsgemäßes Verfahren durchgeführt werden kann.

Figur 1 zeigt eine Ultraschall-Vorrichtung US 1, die mit einer Ultraschall-Sonde 4 verbunden ist, welche einen einstellbaren Erfassungs- und Abbildungsbereich 4a hat. Im Inneren eines Körpers 6 ist ein interessierender Bereich 6a angeordnet, welcher durch die Ultraschall-Vorrichtung US 1 erfasst und in guter Bildqualität dargestellt werden soll. Hierzu wird erfindungsgemäß in dem beispielhaft als Aufnahmevorrichtung verwendeten Kernspintomographen MRI 2 der Körper 6 von der Untersuchung durch die Ultraschall-Vorrichtung US 1 aufgenommen, wobei durch den Kernspintomographen MRI 2 Informationen über Lage und Ausrichtung des interessierenden Bereichs 6a innerhalb des Körpers 6 gewonnen werden.

Beispielsweise kann durch eine Auswerte- oder Recheneinheit PC 7 ermittelt werden, wie weit der interessierende Bereich 6a unterhalb eines oder mehrerer bestimmter O-berflächenpunkte des Körpers 6 liegt.

Die von dem Kernspintomographen MRI 2 gewonnenen Daten werden unmittelbar oder nach Auswertung durch die Einheit PC 7 auf ein Navigationssystem NAV 3 übertragen, welches mit einer bekannten IR-Kamera 3a verbunden ist. Mittels des Navigationssystems NAV 3 kann durch den mit der Ultraschall-Sonde 4 verbundenen Marker 5a und den mit dem Körper 6 verbundenen Marker 5b die Ultraschall-Sonde 4 an einer gewünschten Stelle an oder auf dem Körper 6 positioniert werden.

Die von dem Kernspintomographen MRI 2 gewonnenen Bilddaten des Körpers 6 können durch die Recheneinheit PC 7 so ausgewertet und umgesetzt werden, dass diese als Parameter zum Einstellen der Ultraschallvorrichtung US 1 verwendet werden können, so dass der interessierende Bereich 6a im Inneren des Körpers 6 von der an einer bestimmten Position aufgesetzten Ultraschall-Sonde 4 so erfasst werden kann, dass der interessierende Bereich 6a so im Erfassungs- und Aufnahmebereich 4a der Ultraschall-Sonde 4 liegt, dass dieser interessierende Bereich 6a mit hoher oder optimaler Bildqualität dargestellt werden kann.

Ein Speicher MEM 8 speichert zur Darstellung bestimmter Strukturen wie zum Beispiel Blutgefäße, Gehirn, Muskeln, Knochen besonders vorteilhafte Parameter-Datensätze für die Ultraschall-Vorrichtung US 1, so dass ein Benutzer nur zum Beispiel aus einem vorgegebenen Menü auswählt, dass er beispielsweise Blutgefäße durch die Ultraschall-Vorrichtung US 1 darstellen will, woraufhin die Sonde 4 mit dem Navigationssystem NAV 3 so an dem Körper 6 positioniert wird und die Ultraschall-Vorrichtung US 1 basierend auf den körperspezifischen Aufnahmedaten der Aufnahmevorrichtung MRI 2 und den für Blutgefäße geeigneten Parameterdaten aus dem Speicher MEM 8 so eingestellt wird, dass die in dem Körper 6 verlaufenden Blutgefäße mit guter oder optimierter Bildqualität dargestellt werden.

## Patentansprüche

1. Verfahren zum Einstellen mindestens eines Einstellparameters einer Ultraschall-Vorrichtung (1), wobei ein Objekt (6) durch eine Kemspinresonanzaufnahme (MRI), Computertomographie (CT), PET, SPECT oder Fluroskopie bzw. Röntgenaufnahmen aufgenommen wird und aus den aufgenommenen Bilddaten mindestens ein Einstellwert des mindestens einen Einstellparameters der Ultraschall-Vorrichtung (1) erzeugt wird, wobei die Farbe, die Tiefe, der Tiefenausgleich, der Dynamikbereich, die Kontur, der Fokus, die Bildrate/Zeilendichte, die Frequenz, die Verstärkung, Grauskalen, ein Offset, die Sendeleistung, eine Korrelation, Nachbearbeitungskurven für Graustufen, der Sektorwinkel, die Bildbreite, der Schallkopftyp und/oder eine Vergrößerung als der mindestens eine Einstellparameter der Ultraschall-Vorrichtung (1) verwendet werden und wobei in Abhängigkeit von der Position einer Ultraschall-Sonde (4) und basierend auf den zur Position korrespondierenden Bilddaten der mindestens eine Einstellwert des mindestens einen Einstellparameters der Ultraschall-Vonichtung (1) so gewählt wird, dass zu dieser Position der Ultraschall-Sonde (4) ein möglichst optimales Ultraschall-Bild generiert werden kann.

2. Computerprogramm, welches wenn es in einen Computer geladen wird oder auf einem Computer läuft, das Verfahren nach dem vorhergehenden Anspruch durchführt.

3. Programmspeichermedium oder Computerprogrammprodukt mit dem Programm nach dem vorhergehenden Anspruch.

4. Vorrichtung zum Einstellen mindestens eines Einstellparameters einer Ultraschall-Vorrichtung (1), wobei die Farbe, die Tiefe, der Tiefenausgleich, der Dynamikbereich, die Kontur, der Fokus, die Bildrate/Zeilendichte, die Frequenz, die Verstärkung, Grauskalen, ein Offset, die Sendeleistung, eine Korrelation, Nachbearbeitungskurven für Graustufen, der Sektorwinkel, die Bildbreite, der Schallkopftyp und/oder eine Vergrößerung als der mindestens eine Einstellparameter der Ultraschall-Vorrichtung (1) verwendet werden, zur Erzeugung einer Ultraschall-Aufhahme eines Objekts (6) mit einer Aufnahmevorrichtung (2) zur Aufnahme des Objekts (6) durch eine Kemspinresonanzaufnahme (MRI), Computertomographie (CT), PET, SPECT oder Fluroskopie bzw. Röntgenaufnahmen, einer Auswerteeinheit (7) zur Auswertung der von der Aufnahmevorrichtung (2) gewonnen Bilddaten des Objekts (6) und zur Erzeugung mindestens eines Einstellwertes des mindestens einen Einstellparameters der Ultraschall-Vorrichtung (1), wobei in Abhängigkeit von der Position einer Ultraschall-Sonde (4) und basierend auf den zur Position korrespondierenden Bilddaten der mindestens eine Einstellwert des mindestens einen Einstellparameters der Ultraschall-Vorrichtung (1) so gewählt wird, dass zu dieser Position der Ultraschall-Sonde (4) ein möglichst optimales Ultraschall-Bild generiert werden kann.

5. System mit einer Vorrichtung nach dem vorhergehenden Anspruch und einer Ultraschall-Vorrichtung (1).

6. System nach dem vorhergehenden Anspruch mit einer Schnittstelle oder einem Interface zum Austauschen oder Übertragen von Einstellparametern der Ultraschall-Vorrichtung (1) oder Positionsdaten der Ultraschall-Sonde (4).

## Claims

1. A method for setting at least one setting parameter of an ultrasound device (1), wherein an object (6) is recorded using a nuclear spin resonance recording (MRI), computer tomography (CT), PET, SPECT or fluoroscopy and/or x-ray recordings, and at least one setting value of the at least one setting parameter of the ultrasound device (1) is generated from the recorded image data, wherein the colour, depth, depth gain compensation, dynamic range, contour, focus, image rate/line density, frequency, amplification, grey scales, an offset, the transmitting output, a correlation, post-processing curves for grey scales, the sector angle, image width, type of sound head and/or a zoom are used as the at least one setting parameter of the ultrasound device (1), and wherein depending on the position of an ultrasound probe (4) and based on the image data corresponding to said position, the at least one setting value of the at least one setting parameter of the ultrasound device (1) is selected such that as optimal an ultrasound image as possible can be generated with respect to said position of the ultrasound probe (4).

2. A computer program which, when it is loaded onto a computer or is running on a computer, performs the method according to the preceding claim.

3. A program storage medium or computer program product comprising the program according to the preceding claim.

4. A device for setting at least one setting parameter of an ultrasound device (1), wherein the colour, depth, depth gain compensation, dynamic range, contour, focus, image rate/line density, frequency, amplification, grey scales, an offset, the transmitting output, a correlation, post-processing curves for grey scales, the sector angle, image width, type of sound head and/or a zoom are used as the at least one setting parameter of the ultrasound device (1), for generating an ultrasound recording of an object (6), comprising: a recording device (2) for recording the object (6) using a nuclear spin resonance recording (MRI), computer tomography (CT), PET, SPECT or fluoroscopy and/or x-ray recordings; an evaluating unit (7) for evaluating the image data of the object (6) obtained from the recording device (2) and for generating at least one setting value of the at least one setting parameter of the ultrasound device (1), wherein depending on the position of an ultrasound probe (4) and based on the image data corresponding to said position, the at least one setting value of the at least one setting parameter of the ultrasound device (1) is selected such that as optimal an ultrasound image as possible can be generated with respect to said position of the ultrasound probe (4).

5. A system comprising a device according to the preceding claim and an ultrasound device (1).

6. The system according to the preceding claim, comprising an interface for exchanging or transmitting setting parameters of the ultrasound device (1) or positional data of the ultrasound probe (4).

## Revendications

1. Procédé pour régler au moins un paramètre de réglage d'un dispositif à ultrasons (1), dans lequel on réalise une image d'un objet (6) par résonance magnétique nucléaire (IRM), tomographie assistée par ordinateur (CT), PET, SPECT ou fluoroscopie ou radiographie, et à partir des données d'images obtenues on produit au moins une valeur de réglage du au moins un paramètre de réglage du dispositif à ultrasons (1), la couleur, la profondeur, l'équilibrage des profondeurs, le domaine dynamique, le contour, le foyer, le taux d'image/densité de lignes, la fréquence, l'amplification, l'échelle des grilles, un offset, la puissance d'émission, une corrélation, des courbes de retraitement pour des degrés de grille, l'angle de secteur, la largeur d'image, le type de tête acoustique et/ou un agrandissement étant utilisé en tant que le au moins un paramètre de réglage du dispositif à ultrasons (1), et en fonction de la position d'une sonde à ultrasons (4) et sur la base des données d'image correspondant à la position, la au moins une valeur de réglage du au moins un paramètre de réglage du dispositif à ultrasons (1) étant choisie de manière que pour cette position de la sonde à ultrasons (4) une image ultrasonore aussi optimale que possible puisse être générée.

2. Programme d'ordinateur qui lorsqu'il est chargé dans un ordinateur ou tourne dans un ordinateur met en oeuvre le procédé selon la revendication précédente.

3. Support de mémoire de programme ou produit de programme d'ordinateur avec le programme selon la revendication précédente.

4. Dispositif pour régler au moins un paramètre de réglage d'un dispositif à ultrasons (1), dans lequel la couleur, la profondeur, l'équilibrage des profondeurs, le domaine dynamique, le contour, le foyer, le taux d'image/densité de lignes, la fréquence, l'amplification, l'échelle des grilles, un offset, la puissance d'émission, une corrélation, des courbes de retraitement pour des degrés de grille, l'angle de secteur, la largeur d'image, le type de tête acoustique et/ou un agrandissement est utilisé en tant que le au moins un paramètre de réglage du dispositif à ultrasons (1) pour produire une image ultrasonore d'un objet (6) avec un dispositif d'imagerie (2) pour réaliser une image de l'objet (6) par résonance magnétique nucléaire (IRM), tomographie assistée par ordinateur (CT), PET, SPECT ou fluoroscopie ou radiographie, une unité d'évaluation (7) pour évaluer les données d'image de l'objet (6) obtenues par le dispositif d'imagerie (2) et produire au moins une valeur de réglage du au moins un paramètre de réglage du dispositif à ultrasons (1), en fonction de la position d'une sonde à ultrasons (4) et sur la base des données d'image correspondant à la position, la au moins une valeur de réglage du au moins un paramètre de réglage du dispositif à ultrasons (1) étant choisie de manière que pour cette position de la sonde à ultrasons (4) une image ultrasonore aussi optimale que possible puisse être générée.

5. Système avec un dispositif selon la revendication précédente et un dispositif à ultrasons (1).

6. Système selon la revendication précédente avec une interface pour changer ou transmettre des paramètres de réglage du dispositif à ultrasons (1) ou des données de position de la sonde à ultrasons (4).
